# EUROPEAN PATENT SPECIFICATION

(11) **EP 3 412 623 B1**
(45) Date of publication and mention of the grant of the patent: **03.01.2024**
(21) Application number: 17747600.9
(22) Date of filing: 03.02.2017
(51) Int. Cl.: B08B 9/032

(54) **PURIFICATION METHOD**
REINIGUNGSVERFAHREN
PROCÉDÉ DE PURIFICATION

(30) Priority: 05.02.2016 JP 2016020705
(43) Date of publication of application: 12.12.2018
(73) Proprietor: Suntory Holdings Limited, Osaka-shi, Osaka 530-8203 (JP)
(72) Inventor: TAKAHASHI Tokio, Soraku-gun Kyoto 619-0284 (JP); HIRAYAMA Yuji, Soraku-gun Kyoto 619-0284 (JP); ASHITANI Kengo, Hokuto-shi Yamanashi 408-0316 (JP); NAKAMURA Takuto, Hokuto-shi Yamanashi 408-0316 (JP)
(74) Representative: Vossius & Partner Patentanwälte Rechtsanwälte mbB
(86) International application number: PCT/JP2017/004100
(87) International publication number: WO 2017/135449

(56) References cited:
- EP-A1- 2 821 362
- EP-A1- 2 921 450
- DE-A1- 19 745 538
- JP-A- 2005 131 453
- JP-A- 2007 106 464
- JP-A- 2007 112 519
- JP-A- 2009 136 852
- JP-A- 2014 196 148
- JP-A- 2014 196 148
- US-A1- 2012 018 030

## Description

### TECHNICAL FIELD

The present invention relates to a purifying method having a sterilizing step for sterilizing at least a filling device either periodically or each time when a kind of a liquid product to be filled is to be changed in an aseptic filling system having the filling device configured to fill a predetermined unit filling amount of such liquid product in an individual container.

### DESCRIPTION OF RELATED ART

Container-contained liquid products such as juice-based beverage, tea, water, etc. are manufactured in an aseptic filling system.

The aseptic filling system includes a blending device for blending a plurality of kinds of raw materials at respectively desired proportions, a storage tank for storing liquid product blended by the blending device, a sterilizing device for sterilizing the liquid product discharged from the storage tank, a filling device for filling the liquid product sterilized by the sterilizing device by a predetermined filling amount in an individual container, and so on. The respective devices are fluidly connected to each other via fluid supplying pipes.

The filling device, as disclosed in Patent Document 1, is configured such that a plurality of containers as being supported independently are caused to revolve along a circular orbit, when liquid product is discharged from filling nozzles mounted at tops of a plurality of filling valves disposed along this revolving orbit to be filled in each container.

The respective devices and the liquid supplying pipes used in such aseptic filling system are purified by CIP (Cleaning in Place) technique and/or SIP (Sterilization in Place) technique either periodically or on each occasion of changing the kind of liquid product.

The CIP technique, as shown in Patent Document 2, is implemented e.g. by flushing the respective devices or the liquid supplying pipes with a cleaning solution prepared by adding an alkaline agent or acidic agent to water. With this, e.g. remains of the liquid product can be removed from the respective devices and/or the liquid supplying pipes. The SIP technique is implemented by flushing the CIP purified devices and/or the liquid supplying pipes with steam or cleaning solution. With this, the devices and the liquid supplying pipes can be sterilized to be rendered aseptic.

Patent Document 3 discloses a purifying method comprising a CIP technique and SIP technique.

### RELATED ART DOCUMENTS

### PATENT DOCUMENTS

Patent Document 1: Japanese Unexamined Patent Application Publication No. 2014-093994
Patent Document 2: Japanese Unexamined Patent Application Publication No. 2000-153245
Patent Document 3: Japanese Unexamined Patent Application Publication No. 2014-196148

### SUMMARY OF THE INVENTION

### PROBLEM TO BE SOLVED BY INVENTION

The filling valve included in the filling device has a narrow passage for flowing liquid product and this passage incorporates a valve mechanism and a plurality of rubber packings, etc. Thus, the filling valve has a complicated shape and structure and has a large liquid contact area for contacting liquid product.

Further, at time of stop of operation of the aseptic filling system, a certain amount of liquid product is to stay for a relatively long time inside the filling valve.

Therefore, the smell of the liquid product tends to remain on the passage, the valve mechanism, the rubber packings, etc.

For instance, when the kind of liquid product to be manufactured has been changed, especially when a liquid product having especially strong smell has been changed to another liquid product, the smell of the former liquid product may be transferred to the latter liquid product, thus providing it with a smell or flavor different from its original smell or flavor.

For removing such unwanted smell stuck to the filling device, it is conceivable to extend the period of the CIP technique implementation or to use a strong agent.

However, since the CIP process is effected with temporary stopping of the manufacturing of the container-contained (or bottled) liquid product, such period extension of CIP implementation is undesirable since it invites reduction in the manufacturing efficiency.

Moreover, there was another concern that the smell of the agent used in the CIP process may remain. And, use of strong agent can cause deterioration in the rubber packings included in the filling device.

As described above, in purifying the filling device, the CIP technique may be useful for preventing mixing of remains of the former liquid product in the next liquid product, the technique may fail to provide sufficient effect in the respect of deodorization.

Thus, the primary object of the present invention is to provide a purifying method capable of deodorizing a filling device in a shorter period than the conventional purifying method.

### SOLUTION

For accomplishing the above-noted object, according to a characterizing feature of a purifying method of the present invention, there is provided a purifying method according to claim 1.

As a result of intensive and extensive research effort, the present inventors have discovered that the filling device can be deodorized more effectively with using steam than using a cleaning solution.

Liquid product adhering to and remaining in the passage of the filling valve or intruding into the rubber packings can be desorbed from the passage or the rubber packings by steam supplied at the air supplying step effected prior to or simultaneously with the sterilizing step and the desorbed product together with the smell component present in the gas phase inside the filling device will be discharged from the filling device simultaneously with discharging of the steam from this filling device. Further, although a portion of the steam will condense within the passage, the smell component will be entrapped in this condensate also, and this liquid (condensate) too will be discharged from the filling device together with the rinsing solution or the changed liquid product supplied at the subsequent liquid supplying step.

The sterilizing step is effected after the air supplying step, whereby the filling device is sterilized. Incidentally, the sterilizing step is effected with use of steam.

In the present invention, prior to the air supplying step, there is provided a cleaning step for supplying a cleaning solution to the filling device.

With the above, the air supplying step can be effected after cleaning by the cleaning solution, so that smell or odor of agent used in the cleaning step can be eliminated also.

In the present invention, preferably, the sterilizing step comprises a step of supplying steam to the filling device.

With the above, the sterilization can be implemented with use of steam same as the steam used at the air supplying step. Therefore, there is obtained an advantage of not needing any separate device or unit which would be otherwise needed in case of using a different sterilization method using e.g. hot water, or water solution of weak hypochlorous acid, peracetic acid, caustic soda or nitric acid, etc.

### BRIEF DESCRIPTION OF THE DRAWINGS

Fig. 1 is an explanatory diagram of an aseptic filling system,
Fig. 2 is an explanatory diagram of a filling device,
Fig. 3 is an explanatory diagram of a filling valve,
Fig. 4 is an explanatory diagram of relative evaluation of deodorization effects, and
Fig. 5 is a flowchart of a purifying method.

### DETAILED DESCRIPTION OF THE PREFERRED EMBODIMENTS

Next, an embodiment of a purifying method will be explained with reference to the accompanying drawings.

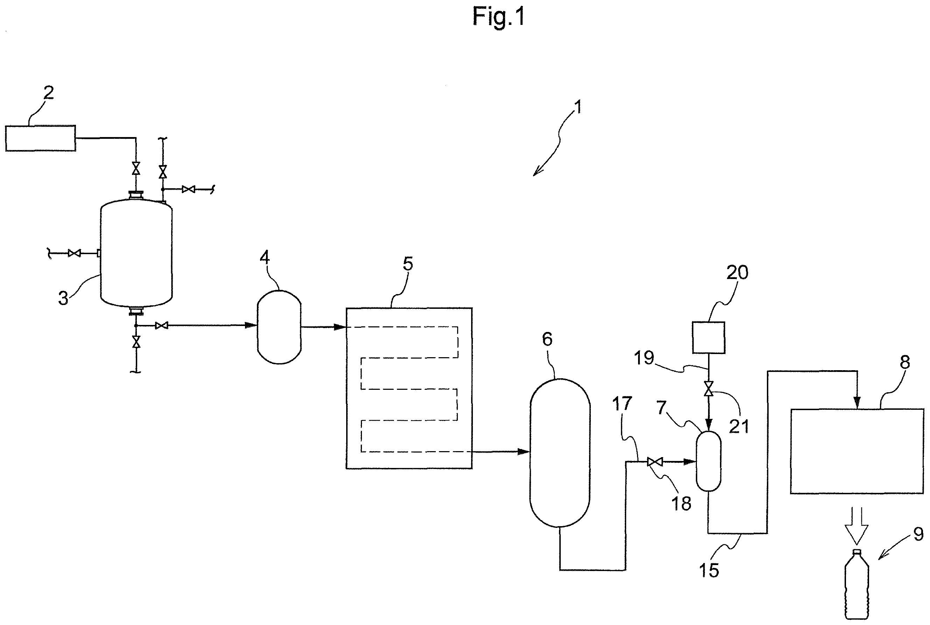

Fig 1 shows an aseptic filling system 1 for manufacturing container-contained liquid product such as juice-based beverage, tea, water, etc.

The aseptic filling system 1 includes a blending device 2, a storage tank 3, a balance tank 4, a sterilizing device 5, a surge tank 6, a head tank 7, a filling device 8, and so on. The respective tanks and the respective devices are fluidly connected to each other via pipes. Each pipe incorporates a valve, a pump (not shown) as needed. The respective components of the aseptic filling system 1 are controlled integrally by a controlling means (not shown). This controlling means or device can be a predetermined controller such as a sequence controller.

The blending device 2 is a device for blending a plurality of kinds of raw materials of liquid product at respective desired proportions.

The storage tank 3 is a tank for storing liquid product blended by the blending device 2.

The balance tank 4 is a tank for temporarily storing an amount of liquid product discharged from the storage tank 3 and then discharging this smoothly to a subsequent stage of the system as needed.

The sterilizing device 5 is a device for sterilizing the liquid product discharged from the balance tank 4 by such a technique as ultra-high temperature heating and is constituted of a heat exchanger, such as a plate type heat exchanger or a shell-and-tube heat exchanger, etc., configured to effect heat exchange between two kinds of liquid having different temperature ranges.

The surge tank 6 is a tank for temporarily storing an amount of liquid product sterilized by the sterilizing device 5; and this surge tank 6 constantly receives pressure with aseptic air at the time of manufacture of liquid product, thus being not affected by any bacterial effect from the outside.

The head tank 7 is a tank for temporarily storing an amount of liquid product discharged from the surge tank 6 and the discharging this smoothly to a subsequent stage of the system as needed.

The filling device 8 is a device for filling a predetermined filling amount of the liquid product discharged from the head tank 7 in an individual container.

Incidentally, the surge tank 6, the head tank 7, the filling device 8 and the pipes interconnecting these are provided with pressure-resistant arrangements.

Next, the filling device 8 will be described in greater details.

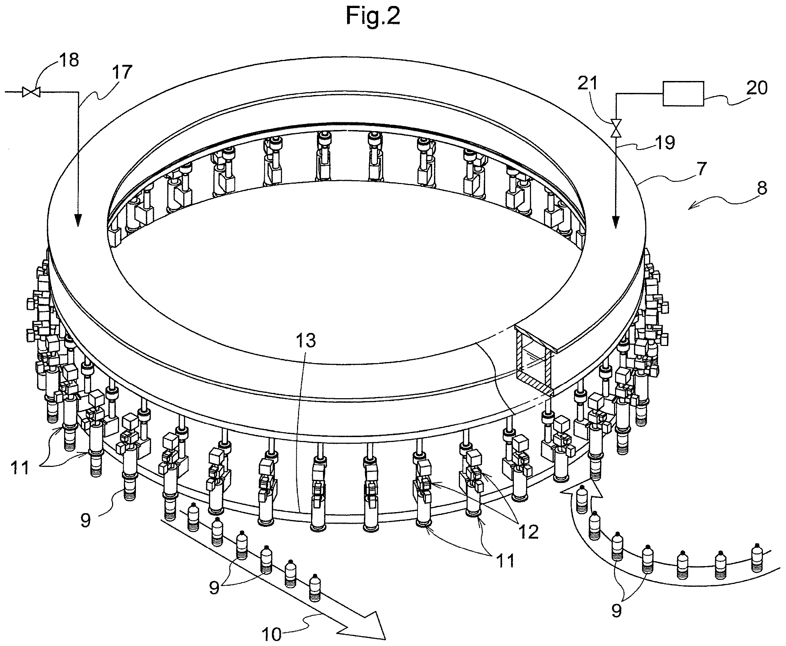

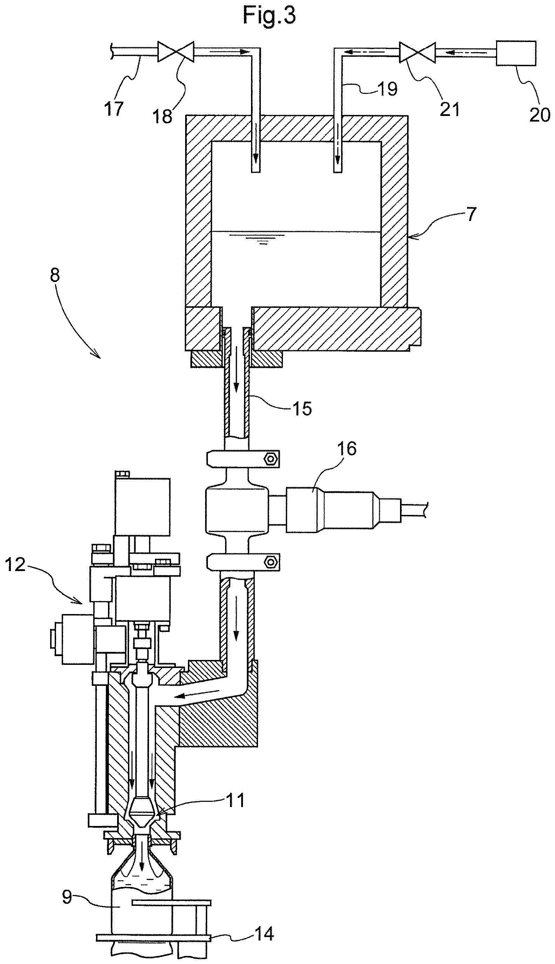

As shown in Fig. 2 and Fig. 3, the filling device 8 includes a star wheel 13 rotatable in a horizontal plane and a plurality of filling valves 12 having respective filling nozzles 11 each of which is configured to fill liquid product in a container 9 such as a PET bottle or a metal can, with the filling valves 12 being disposed with an equal pitch in an annular arrangement around the star wheel 13.

Under the filling valve 12 there is provided a container gripper 14 for gripping the container 9 which has been conveyed by a transporting conveyer 10. An empty container 9 will be cleaned and sterilized by a cleaning device (not shown) and then conveyed by the star wheel 13 to be gripped by the gripper 14.

The filling valve 12 is connected to the head tank 7 via a liquid feeding pipe 15. Incidentally, the liquid feeding pipe 15 incorporates, at a mid position thereof, an electromagnetic flow meter 16.

The head tank 7 stores an amount of liquid product supplied from the surge tank 6 via a liquid feeding pipe 17. The liquid product stored in the head tank 7 will be metered by the electromagnetic flow meter 16 and then supplied to the filling valve 12. Incidentally, the liquid feeding pipe 17 incorporates a valve 18.

The valve 18 is comprised of an electric valve or an electromagnetic valve and is opened/closed under control of the controlling means (not shown). The controlling means controls opening of the valve 18 at the time of manufacture of the container-contained liquid product. With this, the liquid product will be supplied from the surge tank 6 to the head tank 7.

The filling valve 12 includes a narrow passage for flowing the liquid product supplied from the head tank 7 via the liquid feeding pipe 15 to the filling nozzle 11, a valve mechanism incorporated in the passage, a plurality of rubber packings, etc. In association with opening/closing of the valve mechanism, the liquid product will be filled via the filling nozzle 11 into the container 9 gripped by the container gripper 14.

Incidentally, the container 9 filled with the liquid product will be discharged from the container gripper 14 to a transport conveyer 10 to be conveyed to a sealing device (not shown) to be sealed thereby with a cap or the like.

The filling valve 12 has a complicated shape and structure and has also a large contact area for contact with the liquid product. Further, at time of stop of operation of the aseptic filling system 1, an amount of liquid product will stay for a relatively long time inside the filling valve 12. Therefore, the passage, the valve mechanism and the rubber packings included in the filling valve 12 tend to retain the smell of the liquid product.

For deodorization of the filling device 8 configured as described above, in the instant embodiment, there is provided an air supplying mechanism 20 connected to the head tank 7 via an air supplying pipe 19. Incidentally, the air supplying pipe 19 incorporates a valve 21.

The air supplying mechanism 20 is a mechanism having a steam source and a blower and configured to supply steam to the filling device 8 via the air supplying pipe 19.

The valve 21, like the aforementioned valve 18, is constituted of an electric valve or an electromagnetic valve and is opened/closed under control of the controlling means (not shown).

In the aseptic filling system 1, at least at time of periodical maintenance thereof or on each occasion of change of the liquid product to be manufactured, a purifying method according to the present invention is implemented. Incidentally, in the following discussion, the inventive purifying method will be explained in connection with the filling device only. However, the other devices included in the aseptic filling system 1 will also be purified when needed.

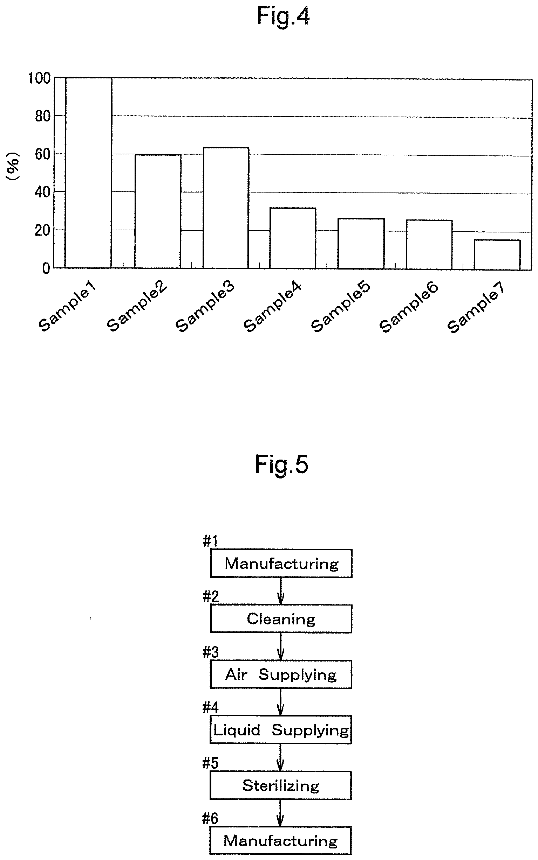

As shown in Fig. 5, this purifying method is useful when a different liquid product (manufacturing step #6) is to be manufactured subsequent to manufacture (manufacturing step #1) of a liquid product having strong smell in particular. Thus, between the manufacturing step #1 and manufacturing step #6, the method includes a cleaning step #2, a deodorizing step (an air supplying step #2, a liquid supplying step #4) and a sterilizing step #5 in this mentioned order.

The cleaning step #2 is effected with stopping of manufacturing of the container-contained liquid product.

The head tank 7 stores an amount of cleaning solution instead of the liquid product. And, this cleaning solution will be supplied to the filling device 8. The cleaning solution to be used can be a cleaning solution prepared by adding an alkaline agent or an acidic agent to water, for example.

In this embodiment, the cleaning step #2 is arranged such that an amount of cleaning solution added with an alkaline agent is supplied for a predetermined period.

By the cleaning step #2, cleaning (flushing) operation of liquid product components remaining on cleaning target faces inside the filling device 8 (removal from the cleaning target faces, dissolution into the cleaning solution, discharge from the filling device 8) is effected.

Still preferably, prior to the cleaning step #2, a preliminary liquid supplying step is effected. With this, rough cleaning of the cleaning target faces inside the filling device 8 will be effected.

Further, preferably, subsequent to the cleaning step #2, a final liquid supplying step is effected. With this, any amount of cleaning solution remaining on the cleaning target faces inside the filling device 8 will be discharged therefrom.

In this way, cleaning of the filling device 8 is carried out.

After completion of the cleaning step #2, the deodorizing step of the filling device 8 is effected.

Incidentally, at the time of completion of the cleaning step #2, the inside of the filling device 8 has become empty.

The deodorizing step includes the air supplying step #3 for supplying steam to the emptied filling device 8 and the liquid supplying step #4 for supplying rinsing solution to the filling device 8 after the air supplying step #3.

At the time of execution of the air supplying step #3, the controlling means closes the valve 18 and opens the valve 21 and operates the air supplying mechanism 20.

At the air supplying step #3, from the air supplying mechanism 20 via the air supplying pipe 19, steam at 101 °C or higher is supplied for a predetermined period. In this, the flow rate per unit period of the steam supplied by the air supplying mechanism 20 is appropriately controlled.

The steam supplied to the head tank 7 is supplied to the filling valve 12 via the liquid feeding pipe 15, whereby liquid product adhering to and remaining on the flow passage of the filling pipe 12 and liquid product entrapped in the rubber packings will be removed from the rubber packings by the steam and then discharged together with the smell component present in the gas phase inside the filling device 8 in association with discharging of the steam via the filling nozzle 11 of the filling valve 12. Further, a portion of the steam will condensate inside the passage, but the smell component will be entrapped in this condensate as well.

Upon completion of the air supplying step #3, the liquid supplying step #4 for the filling device 8 is effected.

At this liquid supplying step #4, rinsing solution is supplied from a rinsing solution supplying means (not shown) and this rinsing solution is supplied via the liquid feeding pipe 15 to the filling valve 12 of the filling device 8. Then, liquid remaining on the cleaning target faces of the filling valve 12 is discharged together with the rinsing solution supplied at the liquid supplying step #4 to the outside via the filling nozzle 11 of the filling valve 12. In this way, deodorization of the filling device 8 is realized.

Upon completion of the liquid supplying step #4, the sterilizing step #5 is effected for the filling device 8 which has been deodorized as described above, so that the filling device 8 is sterilized.

Incidentally, in the instant embodiment, steam is employed in the sterilizing step #5. And, the controlling means controls closing of the valve 18 and opening of the valve 21 and activates the air feeding mechanism 20 at the time of execution of the sterilizing step #5 also.

At the sterilizing step #5, steam having a temperature of 101 °C or higher is supplied for a predetermined period into the head tank 7 from the air supplying mechanism 20 via the air supplying pipe 19. In this, the flow rate per unit period of the steam supplied by the air supplying mechanism 20 is appropriately controlled. In this way, the filling device 8 is sterilized.

The effects of the above-described deodorizing step (the air feeding step #3, the liquid feeding step #4) are verified by respective Experiments 1 and 2 as follows.

In Experiment 1, a test device was configured with reproduction of substantially same liquid contact area, staying liquid amount, etc. of liquid product as the filling device as the actual target of deodorization with use of pipes, rubber packings, etc. Then, to this test device, smell was forcibly transferred with 10-fold concentrated commercially available container-contained flavored liquid product for a predetermined period (about 72 hours). Then, relative evaluations of deodorization effects using various deodorizing agents were conducted. Incidentally, the evaluations were made as sensory tests by a plurality of examiners.

In Experiment 1, Samples 1 through 7 were prepared as testing deodorizing agents.
Sample 1 was a control.
Sample 2 was hot water.
Sample 3 was water added with an alkaline agent only.
Sample 4 was water added with an acidic agent and a commercially available agent A.
Sample 5 was water added with caustic soda and a commercially available agent B.
Sample 6 was water added with caustic soda and a commercially available agent C.
Sample 7 was steam.

Incidentally, the temperatures of Samples 1 through 6 were set to 80 °C. The temperature of sample 7 was set to 130 °C.

As may be apparent from Fig. 4, it can be confirmed that Samples 2 through 7 were more deodorized by Sample 1 which was not deodorized at all. And, especially, with Sample 7, it was confirmed that the deodorization effect was even higher as compared with Samples 2 through 6 using hot water, the agents, etc.

Incidentally, Fig. 4 shows a smell strength comparison of Samples 2-7 relative to the strength of smell evaluated in the sensory experiment of Sample 1 being set as 100.

In Experiment 2, the deodorization period of the purifying method using steam and the deodorization period of the purifying method using the purifying solution added with an alkaline agent were compared.

A test device was configured with reproduction of substantially same liquid contact area, staying liquid amount, etc. of liquid product as the filling device as the actual target of deodorization with use of pipes, rubber packings, etc. Then, to this test device, small was forcibly transferred with a strongly flavored liquid product under a predetermined pressurized state for a predetermined period (about 72 hours). Then, deodorization period when deodorization was effected with steam at a predetermined temperature (101 °C or higher) was determined.

As a result, it was found that the deodorization period of the purifying method using steam was 1/2 to 115 of that of the case of the deodorization by the conventional purifying method using the alkaline agent.

Therefore, it was possible to significantly reduce the suspension period of the aseptic filling system 1 and also to save the amount of cleaning solution for use in deodorization.

As described above, smell can be readily transferred to a rubber packing often used in a filling valve as the smell component of liquid product permeates and gets accumulated therein. And, such smell adhering to the rubber packing can be transferred to a next liquid product inadvertently.

As a result of the present inventor's extensive and intensive research efforts, it was confirmed that such smell adhering to rubber packing can be deodorized efficiently in a short time by using steam, as compared with the case of effecting deodorization by purifying liquid.

In the foregoing embodiment, there was explained the case where between the manufacturing step #1 of the liquid product and the manufacturing step #6 of the further liquid product, there are provided the purifying step #2, the deodorizing step (air supplying step #3, liquid supplying step #4), and the sterilizing step #5 in this mentioned order. According to an embodiment not part of the invention, the air feeding step #3 included in the purifying method can be effected simultaneously with the sterilizing step #5. In this case, since deodorization and sterilization of the filling device 8 can be carried out simultaneously, deodorization of the filling device can be made in an even shorter period than the conventional purifying method.

Further, in the foregoing embodiment, there was explained the case where rinsing solution is supplied to the filling device 8 at the liquid suppling step #4 included in the inventive purifying method. The invention is not limited thereto. At the liquid supplying step #4, new liquid product to be manufactured after switchover can be supplied to the filling device 8. Therefore, in this case, the liquid product manufactured at the manufacturing step #6 will be supplied. By using a liquid product to be manufactured next at the liquid supplying step #4, there is obtained an advantage of no need to prepare a rinsing solution separately.

In the foregoing, there was explained an example in which the air supplying mechanism 20 of steam is connected to the head tank 7 via the air supplying pipe 19. Alternatively, however, with branching of the liquid feeding pipe 15 connecting the head tank 7 and the filling device 8 the air supplying pipe 19 may be connected thereto.

In the foregoing, there was explained an example in which steam is used at the sterilizing step.

However, according to an embodiment not part of the invention, the sterilizing step can employ not only steam, but e.g. water solution of peracetic acid, caustic soda, nitric acid, etc.

In the foregoing, no explanation was given regarding purifying of a system or device other than the filling device of the aseptic filling system 1. However, the purifying step will be effected individually and respectively for the blending device 2, the storage tank 3, the balance tank 4, the sterilizing device 5 and the surge tank 6 also. Incidentally, as regards to the surge tank 6, the sterilizing step too will be effected subsequently to the cleaning step.

It is understood that as regards to the other arrangements also, the embodiment disclosed in this detailed description is only explanatory, and the scope of the present invention is not limited thereto.

### DESCRIPTION OF REFERENCE NUMERALS/MARKS

- 1:: aseptic filling system
- 8:: filling device
- 9:: container

## Claims

1. A purifying method having
a sterilizing step (#5) for sterilizing at least a filling device (8) either periodically or each time when a kind of a liquid product to be filled thereby is to be changed in an aseptic filling system (1) having the filling device (8) configured to fill a predetermined unit filling amount of such liquid product in an individual container (9),
the method comprising, prior to the sterilizing step (#5), the steps of:
a cleaning step (#2) for supplying a cleaning solution to the filling device (8);
a steam supplying step (#3) in which steam is supplied to the filling device (8) which has been emptied of the liquid product; and
a liquid supplying step (#4) for supplying, after the steam supplying step (#3), a rinsing solution or a new liquid product after product change to the filling device (8),
wherein the sterilizing step (#5) comprises a step of supplying steam to the filling device (8).

2. The purifying method of claim 1, wherein prior to the cleaning step (#2) a preliminary liquid supplying step is effected.

3. The purifying method of claim 1 or 2, wherein subsequent to the cleaning step (#2), a final liquid supplying step is effected.

## Patentansprüche

1. Ein Reinigungsverfahren, enthaltend
einen Sterilisierungsschritt (#5) zum Sterilisieren mindestens einer Abfüllvorrichtung (8) entweder periodisch oder jedes Mal, wenn eine Art eines flüssigen Produkts, das dadurch abgefüllt werden soll, gewechselt werden soll, in einem aseptischen Abfüllsystem (1) mit der Abfüllvorrichtung (8), die konfiguriert ist, um eine vorbestimmte Einheitsfüllmenge eines solchen flüssigen Produkts in einen einzelnen Behälter (9) abzufüllen,
wobei das Verfahren vor dem Sterilisierungsschritt (#5) die Schritte umfasst:
einen Reinigungsschritt (#2) zum Zuführen einer Reinigungslösung zu der Abfüllvorrichtung (8);
einen Dampfzuführungsschritt (#3), in dem Dampf der Abfüllvorrichtung (8) zugeführt wird, aus der das flüssige Produkt entleert wurde; und
einen Flüssigkeitszuführungsschritt (#4), um nach dem Dampfzuführungsschritt (#3) der Abfüllvorrichtung (8) eine Spüllösung oder ein neues flüssiges Produkt nach einem Produktwechsel zuzuführen,
wobei der Sterilisierungsschritt (#5) einen Schritt des Zuführens von Dampf zu der Abfüllvorrichtung (8) umfasst.

2. Das Reinigungsverfahren nach Anspruch 1, wobei vor dem Reinigungsschritt (#2) ein vorausgehender Flüssigkeitszuführungsschritt durchgeführt wird.

3. Das Reinigungsverfahren nach Anspruch 1 oder 2, wobei anschließend an den Reinigungsschritt (#2) ein abschließender Flüssigkeitszuführungsschritt durchgeführt wird.

## Revendications

1. Méthode de purification ayant
une étape de stérilisation (#5) pour stériliser au moins un dispositif de remplissage (8) soit périodiquement soit à chaque fois qu'un type de produit liquide devant être introduit par celui-ci doit être changé dans un système de remplissage aseptique (1) ayant le dispositif de remplissage (8) configuré pour introduire une quantité de remplissage unitaire prédéterminée de ce produit liquide dans un récipient individuel (9),
la méthode comprenant, avant l'étape de stérilisation (#5), les étapes suivantes :
une étape de nettoyage (#2) pour délivrer une solution de nettoyage au dispositif de remplissage (8) ;
une étape de délivrance de vapeur (#3) dans laquelle de la vapeur est délivrée au dispositif de remplissage (8) qui a été vidé du produit liquide ; et
une étape de délivrance de liquide (#4) pour délivrer au dispositif de remplissage (8), après l'étape de délivrance de vapeur (#3), une solution de rinçage ou un nouveau produit liquide après le changement de produit,
dans laquelle l'étape de stérilisation (#5) comprend une étape de délivrance de vapeur au dispositif de remplissage (8).

2. Méthode de purification selon la revendication 1, dans laquelle, avant l'étape de nettoyage (#2), une étape de délivrance de liquide préliminaire est effectuée.

3. Méthode de purification selon la revendication 1 ou 2, dans laquelle, après l'étape de nettoyage (#2), une étape de délivrance de liquide finale est effectuée.
